# EUROPEAN PATENT SPECIFICATION

(11) **EP 0 501 557 B1**
(45) Date of publication and mention of the grant of the patent: **12.10.1994**
(21) Application number: 92200441.1
(22) Date of filing: 17.02.1992
(51) Int. Cl.: C07K 7/08, G01N 33/576, A61K 39/29, C12Q 1/68, C12Q 1/70

(54) **Peptides immunochemically reactive with antibodies directed against hepatitis Non-A, Non-B virus**
Mit Antikörper gegen Hepatitis non-A, non-B-Virus immunochemische reaktive Peptide
Peptides, immunochimiquement réactives avec anticorps contre le virus de l'hépatite non-A, non-B

(30) Priority: 01.03.1991 EP 91200430
(43) Date of publication of application: 02.09.1992
(73) Proprietor: Akzo Nobel N.V., 6824 BM Arnhem (NL)
(72) Inventor: Habets, Winand Johannes Antonius, NL-5232 JB Den Bosch (NL)
(74) Representative: Hermans, Franciscus G.M.

(56) References cited:
- EP-A- 0 388 232
- WO-A-90/14436
- JAPAN. J. EXP. MED. vol. 60, no. 3, 1990, pages 167 - 177; H. OKAMOTO: 'The 5' terminal sequence of the Hepatitis C Viral Genome'
- JAPAN. J. EXP. MED vol. 60, no. 4, 1990, pages 223 - 233; H. OKAMOTO: 'Enzyme-linked Immunosorbent Assay'
- PEPTIDE CHEMISTRY 1990, pages 211 - 214; E. MUNEKATA: 'Epitope mapping of Hepatitis C'
- HEPATOLOGY vol. 14, no. 1, 1991, pages 595 - 600; G. INCHAUSPE: 'Use of conserved sequences from Hepatitis C virus'
- BIOCHEM. BIOPHYS. RES. COMM. vol. 172, no. 2, 1990, pages 511 - 516; K. MURAISO: 'A structural protein of Hepatitis C virus'
- J. GEN. VIROL. vol. 71, 1990, pages 3027 - 3033; K. TAKEUCHI: 'The putative nucleocapsid and envelope genes of Hepatitis C virus'

## Description

The invention relates to peptides which react immunochemically with antibodies directed against hepatitis Non-A, Non-B virus (NANBH-virus).

The invention further relates to a method for the detection of NANBH or anti-NANBH in a test fluid, and to an immunochemical reagent and a test kit to be used when applying the said detection method.

Non-A, Non-B hepatitis, which may or may not be caused by Hepatitis C Virus (HCV), is a transmissible disease or family of diseases shown to be virus-induced. It can be distinguished from other forms of viral-associated liver diseases, including that caused by the known hepatitis viruses, i.e., hepatitis A virus (HAV), hepatitis B virus (HBV), and delta hepatitis virus (HDV), as well as the hepatitis induced by cytomegalovirus (CMV) or Epstein-Barr virus (EBV). NANBH was first identified in transfused individuals. Transmission from man to chimpanzee and serial passage in chimpanzees provided evidence that NANBH is due to a transmissible infectious agent or agents.

Epidemiologic evidence is suggestive that there may be three types of NANBH: the water-borne epidemic type; the blood or needle associated type; and the sporadically occurring (community acquired) type. However, the number of agents which may be the causative of NANBH is unknown.

Clinical diagnosis and identification of NANBH has been accomplished primarily by exclusion of other viral markers. Among the methods used to detect putative NANBH antigens and antibodies are agar-gel diffusion, counter-immunoelectrophoresis, immunofluorescence microscopy, immune electron microscopy, radioimmunoassay, and enzyme-linked immunosorbent assay. However, none of these assays has proved to be sufficiently sensitive, specific, and reproducible to be used as a diagnostic test for NANBH.

However, for the development of a specific and sensitive method to enable a reliable diagnosis to be made in various phases of the infection with NANBH it is of great importance to identify immuno-dominant viral epitopes of this type. The reactivity of a 36 peptide from the HCV core region with sera from HCV patients in an ELISA system has been described by Munekata et al. Peptide Chemistry, 211, 1990.

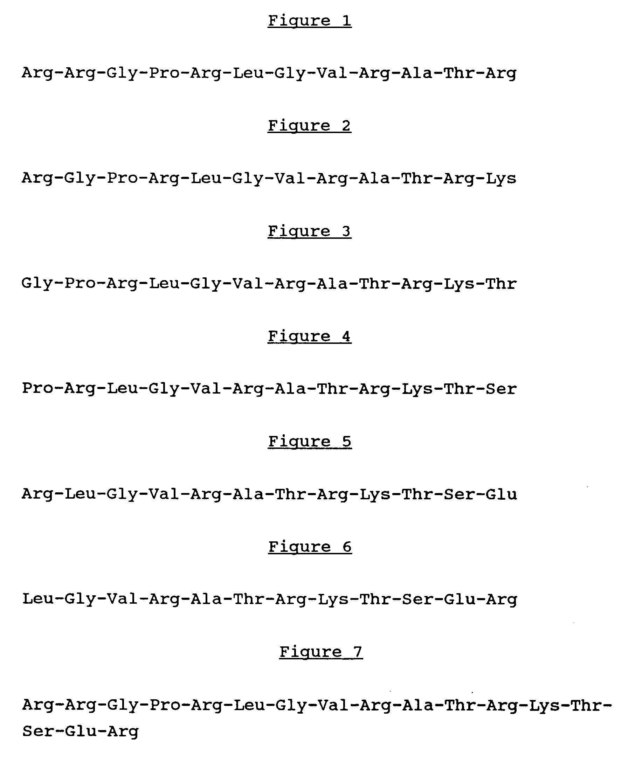

Six peptides has now been found with 12 amino acids and amino acid sequences as shown in figures 1-6, as described hereunder, which are exceptionally immunochemically reactive with NANBH antibodies. These sequences cover the amino acid sequence mentioned in Figure 7.

### Figure 1

Arg-Arg-Gly-Pro-Arg-Leu-Gly-Val-Arg-Ala-Thr-Arg

### Figure 2

Arg-Gly-Pro-Arg-Leu-Gly-Val-Arg-Ala-Thr-Arg-Lys

### Figure 3

Gly-Pro-Arg-Leu-Gly-Val-Arg-Ala-Thr-Arg-Lys-Thr

### Figure 4

Pro-Arg-Leu-Gly-Val-Arg-Ala-Thr-Arg-Lys-Thr-Ser

### Figure 5

Arg-Leu-Gly-Val-Arg-Ala-Thr-Arg-Lys-Thr-Ser-Glu

### Figure 6

Leu-Gly-Val-Arg-Ala-Thr-Arg-Lys-Thr-Ser-Glu-Arg

### Figure 7

Arg-Arg-Gly-Pro-Arg-Leu-Gly-Val-Arg-Ala-Thr-Arg-Lys-Thr-
Ser-Glu-Arg

The invention also includes fragments of the said peptides which are still immunochemically reactive with NANBH-antibodies and also polypeptides which contain the said peptide as an essential constituent, as well as functional derivatives thereof.

The dodecapeptides with amino acid sequence according to Figure 1 and Figure 5 respectively are preferred as peptide according to the invention.

The invention also relates to an immunochemical reagent, which reagent contains at least one of the peptides according to the invention.

The invention also comprises a method for the detection of antibodies directed against NANBH in a test fluid, whereby one or more of the peptides according to the invention are used.

The invention also relates to a method for the detection of NANBH in a test fluid, using one or more of the peptides according to the invention.

Finally, the invention relates to a test kit to be used for carrying out an immuno-assay, this test kit containing at least one immunochemical reagent according to the invention.

Furthermore it is within the scope of this invention to use the new nucleotide sequence or part(s) thereof coding for the amino acid sequence(s) according to the invention, so-called primers, as basis of a test to detect NANBH DNA or RNA by a nucleic acid amplification technique for instance the polymerase chain reaction (PCR) or the nucleic acid sequence based amplification (NASBA), as described in USP 4,683,202 and EP 329,822, respectively.

A part of the invention includes a test amplification kit for carrying out an amplification and detection method described above.

Moreover, a peptide or fragment thereof according to the invention can be used in suitable pharmaceutical dosage forms in the prevention and/or treatment of NANB Hepatitis-disease. The preparation of vaccines thus obtained using such a peptide or fragment thereof as active ingredients, can be accomplished by one skilled in the art.

The peptides mentioned above are particularly suitable for use in a diagnostic method for the determination of the presence of NANBH or NANBH-antibodies in a test fluid.

In addition to the above-mentioned peptides, functional derivatives of these peptides are also considered to belong to the peptides according to the present invention. Functional derivatives of the peptides are meant to include:
(a) acid addition salts of the peptides;
(b) amides of the peptides and specifically the derivatised C-terminal amides;
(c) esters and specifically C-terminal esters and
(d) modified N-acyl derivatives, specifically modified N-terminal acyl derivatives and in particular modified N-acetyl derivatives.

In contrast to the natural NANBH, the peptides according to the invention have the great advantage that these are of a safe non-infectious origin. Furthermore, the present peptides have a particular high affinity to NANBH antibodies, which renders said peptides extremely suitable for use in diagnostic test methods.

The preparation of the peptides according to the invention is effected by means of one of the known organic chemical methods for peptide synthesis or with the aid of recombinant DNA techniques. This latter method involves the preparation of the desired peptide by means of bringing to expression a recombinant polynucleotide with a polynucleotide sequence which is coding for one or more of the peptides in question in a suitable micro-organism as host.

The organic chemical methods for peptide synthesis are considered to include the coupling of the required amino acids by means of a condensation reaction, either in homogeneous phase or with the aid of a so-called solid phase.

The condensation reaction can be carried out as follows:
a) condensation of a compound (amino acid, peptide) with a free carboxyl group and protected other reactive groups with a compound (amino acid, peptide) with a free amino group and protected other reactive groups, where one of the protecting groups also may be a (derivatised) solid support, in the presence of a condensation agent,
b) condensation of a compound (amino acid, peptide) with an activated carboxyl group and free or protected other reaction groups with a compound (amino acid, peptide) with a free amino group and free or protected other reactive groups, where one of the protecting groups also may be a (derivatised) solid support.

Activation of the carboxyl group can take place, inter alia, by converting the carboxyl group to an acid halide, azide, anhydride, imidazolide or an activated ester, such as the N-hydroxy-succinimide, N-hydroxy-benzotriazole, p-nitrophenyl, 3,4-dihydro-3-hydroxy-4-oxo-1,2,3-benzotriazine (ODhbt) or pentafluorophenyl (OPfp) ester.

The most common methods for the above condensation reactions are: the carbodiimide method, the BOP method [benzotriazolyloxytris (dimethyl-amino) phosphonium hexafluoro phosphate], the TBTU method [2-(1H-benzotriazol-1-yl)-1,1,3,3-tetramethyluronium tetrafluoroborate, the azide method, the mixed anhydride method and the method using activated esters, such as described in The Peptides, Analysis, Synthesis, Biology Vol. 1-3 (Ed. Gross, E. and Meienhofer, J.) 1979, 1980, 1981 (Academic Press, Inc.).

Particularly suitable solid phases are, for example, the p-alkoxybenzyl alcohol resin (4-hydroxy-methyl-, phenoxy-methyl-copolystrene-1% divinylbenzene resin), described by Wang (1974) J. Am. Chem. Soc. 95, 1328 and similarly functionalized co-polymers of N,N-dimethylacrylamide, acryloylsarcosine methylester and bisacryloylethylenediamine supported by a matrix of an inert macroporous kieselguhr described by Atherton (1981) J. Chem. Soc., Chem. Comm. 1151. After synthesis the peptides can be split from this solid phase under mild conditions. Other suitable supports are derivatised cross-linked polystyrene, polyethylene or polypropylene rods as described by Geysen, P.N.A.S., 81, 3998 (1984) and P.N.A.S. 82, 178 (1985).

After synthesis of the desired amino acid sequence, either in solution or on a solid support, the protective groups can be split off by various conventional methods, depending on the nature of the particular group, for example with the aid of trifluoro-acetic acid or by mild reduction, for example with hydrogen and a catalyst, such as palladium, treatment with a base as for example piperidine or hydroxide ions, or with HBr in glacial acetic acid.

If the peptide is synthesized on a solid support from which it can be detached this can be achieved, depending on the type of linker, for example, with trifluoro-acetic acid, trifluoromethanesulphonic acid or with methanesulphonic acid dissolved in trifluoroacetatic acid, transesterification with a lower alcohol, preferably methanol or ethanol, in which case a lower alkyl ester of the peptide is formed directly. Likewise, splitting with the aid of ammonia or hydrazine hydrate gives the amide or, respectively, the hydrazide of a peptide according to the invention.

The reactive groups which may not participate in the condensation reaction are, as stated, effectively protected by groups which can be removed again very easily by hydrolysis with the aid of acid, base, or reduction. Thus, a carboxyl group can be effectively protected by, for example, esterification with methanol, ethanol, tertiary butanol, benzyl alcohol or p-nitrobenzyl alcohol and amines linked to solid supports.

Groups which can effectively protect an amino group are the ethoxycarbonyl, benzyloxycarbonyl, t-butoxycarbonyl, 9-fluorenyl-methoxycarbonyl(Fmoc) or p-methoxy-benzyloxycarbonyl group, or an acid group derived from a sulphonic acid, such as the p-toluenesulphonyl, pentamethylbenzene sulfonyl (Pms), 4-methoxy-2,3,6-trimethylbenzene sulfonyl (Mtr) or 1,2,5,7,8-pentamethylchroman-6-sulfonyl (Pmc) group, but other groups can also be used, such as substituted or unsubstituted aryl or aralkyl groups, for example benzyl and triphenylmethyl, or groups such as orthonitrophenyl-sulphenyl and 2-benzoyl-1-methylvinyl.

A more extensive account of possible protecting groups can be found in The Peptides, Analysis, Synthesis, Biology Vol. 1-9 (Eds. Gross, Udenfriend and Meienhofer) 1979-1987 (Academic Press, Inc.).

Preparation of the abovementioned peptides according to the invention using the "solid phase" is for example described in J. Amer. Chem. Soc. 85, 2149 (1963) and Int. J. Peptide Protein Res. 35, 161-214 (1990). The coupling of the amino acids of the peptide to be prepared usually starts from the carboxyl end side. For this method a solid phase is needed on which there are reactive groups or on which such groups can be introduced. This can be, for example, a copolymer of benzene and divinylbenzene with reactive chloromethyl groups, or another suitable polymeric solid phase rendered reactive with hydroxymethyl or amine functions.

As already indicated above, the peptide according to the invention can likewise be prepared with the aid of recombinant DNA techniques. This possibility is of importance particularly when the peptide is incorporated in a repeating sequence ("in tandem") or when the peptide can be prepared as an essential constituent of a (much larger) protein or polypeptide. This type of preparation of the peptide therefore likewise falls within the scope of the invention. For this purpose, as a constituent of a recombinant DNA, a polynucleotide is used which codes for the peptide according to the invention and which, furthermore, is substantially free from polynucleotide segments, which in the naturally occurring NANBH genome flank the polynucleotide sequence indicated above.

A polynucleotide of this type, which is coding for the peptide according to the invention, and a recombinant DNA in which this polynucleotide is incorporated likewise fall within the scope of the invention.

Without this specifically being incorporated in the claims, it is self-evident that one or more amino acids in the peptides according to the invention can be replaced by other amino acids.

The peptides or fragments thereof prepared and described above are used to produce antibodies, both polyclonal and monoclonal. Monoclonal antibodies directed against peptides according to the invention can be readily produced by one skilled in the art.

Making monoclonals by hybridomas is well known. Cell fusion, immortal antibody-producing cell lines can be created while also other techniques are available such as direct transformation of B-lymphocytes with oncogenic DNA or transfection with Epstein-Barr Virus.

Antibodies, both monoclonal and polyclonal, directed against peptides according to the invention are very suitable in diagnosis, while those antibodies which are neutralizing are very useful in passive immunotherapy. Especially monoclonal antibodies may be used to raise anti-idiotype antibodies. Techniques for raising anti-idiotype antibodies are known in the art.

Said anti-idiotype antibodies are also useful for prevention and/or treatment of Non-A, Non-B Hepatitis, as well as for the elucidation of important epitopic regions of NANBH-antigens.

The "immunochemical reagent" according to the invention usually consists of one or more peptides according to the invention and a suitable support or a labelling substance.

Supports which can be used are, for example, the inner wall of a microtest well or a cuvette, a tube or capillary, a membrane, filter, test strip or the surface of a particle such as, for example, a latex particle, an erythrocyte, a dye sol, a metal sol or metal compound as sol particle, a carrier protein such as BSA or KLH.

Labelling substances which can be used are, inter alia, a radioactive isotope, a fluorescent compound, an enzyme, a dye sol, metal sol or metal compound as sol particle.

In a method for the detection of antibodies directed against NANBH in a test fluid, an immunochemical reagent according to the invention is brought into contact with the test fluid. After which, the presence of immune complexes formed between the peptide and antibodies in the test fluid is detected, and by this detection the presence of NANBH antibodies in the test fluid is known and can be determined quantitatively.

Depending on the nature and further characteristics of the immunochemical reagent the immunochemical reaction that takes place is a socalled sandwich reaction, an agglutination reaction, a competition reaction or an inhibition reaction.

For the detection of NANBH in a test fluid an immunochemical reagent according to the invention is brought into contact with the test fluid and anti-NANBH after which the presence of immune complexes formed is detected and, from this, the presence of NANBH in a test fluid can be determined.

A particularly suitable method for the detection of NANBH in a test fluid is based on a competition reaction between a peptide according to the invention provided with a labelling substance and a NANBH antigen (present in the test fluid) whereby the peptide and the antigen are competing with the antibody directed against NANBH attached to a solid support.

A test kit according to the invention comprises, as an essential constituent, an immunochemical reagent as described above. Carrying out a sandwich reaction for the detection of NANBH antibodies the test kit may comprise, for instance, the peptide according to the invention coated to a solid support, for example the inner wall of a microtest well, and either a labelled peptide according to the invention or a labelled anti-antibody.

In carrying out a competition reaction for detection of NANBH antibodies, the test kit may comprise the peptide according to the invention coated to a solid support and a labelled antibody directed against NANBH preferably a monoclonal antibody directed against said peptide.

In an agglutination reaction the test kit comprises an immunochemical reagent which consists of a peptide according to the invention coated to particles or sols.

A test kit for the detection of NANBH antigen comprises for example a labelled peptide according to the invention and an antibody directed against NANBH, which is coated to a solid support.

### Example I

The dodecapeptides with the sequences as shown in figures 1-6 were prepared by stepwise solid phase peptide synthesis. The synthesis was carried out using a Milligen/Biosearch 9050 automated peptide synthesizer or a Labortec SP640 semi-automatic peptide synthesizer, employing a functionalized polymer of copoly(dimethylacrylamide-bisacryloylethylene diamineacryloylsarcosine methylester) supported by macro-porous kieselguhr or a p-benzyloxybenzyl alcohol resin (Wang-resin; 0.6-0.7 mmoles/g, Bachem AG, Switzerland) and N^{α}-Fmoc-protected (Fmoc, 9-fluorenyl-methyloxycarbonyl) amino acids.

For instance the synthesis of the peptide of figure 1 started by the coupling of Fmoc-Arg(Pmc)-OH to the resin using DCC (dicyclohexyl-carbodiimide, 1 equivalent), HOBt (1-hydroxybenzo-triazole, 2 equivalents) and DMAP (N,N-dimethylamino-pyridine, 1 equivalent) in DMF-dichloromethane (1:1, vol/vol) at 4 ^{o}C for 18 hours. Unreacted alcohol functions on the resin were then blocked by benzoylation using benzoylchloride-pyridine for 2 hours.

The resulting Fmoc-Arg(Pmc)-resin (0.38 mmol/g) was successively treated three times with 20% piperidine in DMF for 6 min, in order to remove the Fmoc-group. The protected dodecapeptide was then prepared on the H-Arg(Pmc)-resin by successive coupling steps of the Fmoc-amino acids, as dictated by the amino acid sequence. The following side chain protecting groups were used: -Pmc (2,2,5,7,8-pentamethylchroman-6-sulfonyl-) for Arg; -tBu (tert.butyl-) for Thr.

Each coupling step was performed using 3 equivalents each of Fmoc-amino acid, BOP (= benzotriazolyloxy-tris(dimethylamino)-phosphonium hexafluorophosphate), HOBt and 4,5 equivalents of N-methylmorpholine in 12-15 ml of DMF per gram resin for 45 min, followed by 3 cycles of washings (one min each) with DMF and ethanol. Completeness of the coupling reaction was monitored by the ninhydrin test of Kaiser (Anal. Biochem. 34, 595-598, 1970). A positive ninhydrin reaction was observed following coupling of Ala¹⁰, Arg⁹, Val⁸, Leu⁶, Arg⁵ and Pro³. In each case the coupling reaction was repeated once using one equivalent of corresponding Fmoc-amino acid, BOP, HOBt and N-methylmorpholine for 30 min. Any remaining free amino groups were then blocked by acetylation using acetic anhydride-DMF (5:95; vol/vol) for 10 min and subsequent washings with DMF and ethanol (1 min each), respectively.

After each synthesis cycle the N^{α}-Fmoc-protecting group was removed by treatment with 25% piperidine in DMF as described above.

After completion of the synthesis, the resulting fully protected dodecapeptide resin was treated in a mixture of trifluoro acetic acid - water - phenol - thioanisole - ethanedithiol (82:5:5:5:2,5 vol/vol) for 18 hours at room temperature, in order to effect release of the peptide from the resin with simultaneous removal of all protecting groups. The crude peptide was isolated following precipitation upon addition of the reaction mixture to diethyl ether. The dodecapeptide was purified by HPLC on C₁₈-silica using a gradient of acetonitrile in 0.1 M phosphate buffer at pH 2.1.

### Example II

In a corresponding manner were prepared the dodecapeptides according to figures 2-6.

### Example III

The dodecapeptides with the amino acid sequence as shown in figures 1-6 were individually dissolved to 7.5 µg/ml in 100 mM phosphate buffer pH 8.0. Microtiter plates were pre-treated with 0.2% glutar aldehyde in phosphate buffer pH 5.0 at 135 µl per well for 4 h at room temperature under continuous shaking. Plates were then emptied and individual aliquots of 135 µl of the above peptide solutions was given to each well. Binding of the peptides to the microtiter plate was allowed to proceed for 3 h at 37 ^{o}C. The plates were frozen and stored overnight at -20 ^{o}C .

Subsequently the plates were thawed and emptied, and residual binding sites were blocked with a solution of 0.05% Tween 20^{(R)} in 0.2 M Tris pH 7.4/0.2 M NaCl for 5 min. at room temperature. Plates were then washed once with 0.2 M Tris pH 7.4/0.2 M NaCl and twice with 0.04 M Tris pH 7.4, at 250 µl per well. For the determination of antibodies specific for Non-A, Non-B hepatitis, the serum sample was diluted in sample diluent (phosphate buffered saline (PBS)/20% normal goat serum/1% Triton X100) pipetted into the well (100 µl per well) and incubated for 1 h at 37 ^{o}C. After washing the wells with PBS/0.05% Tween 20^{(R)} the bound human antibodies were detected with goat anti-human immunoglobulin labeled with peroxidase (100 µl per well, 1 h at 37 ^{o}C) diluted in sample diluent. The plates were washed 4 times with PBS/0.05% Tween 20^{(R)}. TMB was added (100 µl per well) as a substrate for the peroxidase enzyme and the reaction was allowed to proceed for 30 min. at room temperature. The reaction was stopped by adding 100 µl 2M H₂SO₄ to each well. The yellow color was read at 450 nm in an Organon Teknika microelisa reader.

With sera from patients with Non-A, Non-B hepatitis, extinctions ranging from 0.6 to 0.8 were measured whereas the mean extinction of 20 normal human sera was 0.250 (standard deviation 0.047). As a control, the procedure was repeated with unrelated dodecapeptides. In all cases tested (n=400), no significant differences were observed in the extinctions obtained with normal human sera and serum samples from patients with NANBH.

## Claims (Claims for the following Contracting State(s): AT, BE, CH, DE, DK, FR, GB, IT, LI)

1. Peptide, immunochemically reactive with anti-HCV antibodies, having the amino acid sequence as indicated in figure 7.

2. Peptide, immunochemically reactive with anti-HCV antibodies, having the amino acid sequence as indicated in figure 1.

3. Immunochemical reagent comprising the peptide of claim 2.

4. Peptide, immunochemically reactive with anti-HCV antibodies, having the amino acid sequence as indicated in figure 5.

5. Immunochemical reagent comprising the peptide of claim 4.

6. Method for the detection of antibodies directed against HCV in a test fluid, characterized in that an immunochemical reagent according to claim 3 or 5 is brought into contact with the test fluid and the presence of immune complexes formed between the peptide and the antibodies in the test fluid is detected.

7. Method for the detection of HCV in a test fluid, characterized in that an immunochemical reagent according to claim 3 or 5 is brought into contact with the test fluid and anti-HCV antibodies, after which the presence of immune complexes formed is detected and the presence of HCV in the test fluid is determined.

8. Test-kit for the detection of anti-HCV antibodies, according to the method of claim 6 or 7.

## Claims (Claims for the following Contracting State(s): ES, GR)

1. Method for the production of a peptide, immunochemically reactive with anti-HCV antibodies, having the amino acid sequence as indicated in figure 7, using one of the known organic chemical methods for peptide synthesis or using recombinant DNA techniques.

2. Method for the production of a peptide, immunochemically reactive with anti-HCV antibodies, having the amino acid sequence as indicated in figure 1, using one of the known organic chemical methods for peptide synthesis or using recombinant DNA techniques.

3. Method for the production of a peptide, immunochemically reactive with anti-HCV antibodies, having the amino acid sequence as indicated in figure 5, using one of the known organic chemical methods for peptide synthesis or using recombinant DNA techniques.

4. Method for the detection of antibodies directed against HCV in a test fluid, characterized in that an immunochemical reagent comprising a peptide, having the amino acid sequence as indicated in any of figures 1, 5 and 7 is brought into contact with the test fluid and the presence of any immune complexes formed between the peptide and the antibodies in the test fluid is detected.

5. Method for the detection of HCV in a test fluid, characterized in that an immunochemical reagent comprising a peptide, having the amino acid sequence as indicated in any of figures 1, 5 and 7 is brought into contact with the test fluid and anti-HCV antibodies, after which the presence of immune complexes formed is detected and the presence of HCV in the test fluid is determined.

## Patentansprüche (Patentansprüche für folgende(n) Vertragsstaat(en): AT, BE, CH, DE, DK FR, GB, IT, LI)

1. Peptid mit der in Figur 7 gezeigten Aminosäurensequenz, das mit anti-HCV-Antikörpern immunochemisch reaktiv ist.

2. Ein mit anti-HCV-Antikörpern immunochemisch reaktives Peptid, das die in Figur 1 gezeigte Aminosäurensequenz besitzt.

3. Immunochemisches Reagenz, das das Peptid gemäss Anspruch 2 umfasst.

4. Ein mit anti-HCV-Antikörpern immunochemisch reaktives Peptid, das die in Figur 5 gezeigte Aminosäurensequenz besitzt.

5. Immunochemisches Reagenz, das das Peptid gemäss Anspruch 4 umfasst.

6. Verfahren zum Nachweis gegen HCV gerichteter Antikörper in einer Testflüssigkeit, dadurch gekennzeichnet, dass ein immunochemisches Reagenz gemäss Anspruch 3 oder 5 mit der Testflüssigkeit in Kontakt gebracht wird und die Anwesenheit von Immunkomplexen, die zwischen dem Peptid und den Antikörpern in der Testflüssigkeit gebildet werden, nachgewiesen wird.

7. Verfahren zum Nachweis von HCV in einer Testflüssigkeit, dadurch gekennzeichnet, dass ein immunochemisches Reagenz gemäss Anspruch 3 oder 5 mit der Testflüssigkeit und anti-HCV-Antikörpern in Kontakt gebracht wird, wonach die Anwesenheit von gebildeten Immunkomplexen nachgewiesen und die Anwesenheit von HCV in der Testflüssigkeit bestimmt wird.

8. Testgarnitur zum Nachweis von anti-HCV-Antikörpern, gemäss dem Verfahren von Anspruch 6 oder 7.

## Patentansprüche (Patentansprüche für folgende(n) Vertragsstaat(en): ES, GR)

1. Verfahren zur Herstellung eines Peptids mit der in Figur 7 gezeigten Aminosäurensequenz, das mit anti-HCV-Antikörpern immunochemisch reaktiv ist, unter Anwendung der bekannten organisch chemischen Verfahren zur Peptidsynthese oder unter Anwendung rekombinanter DNA-Techniken.

2. Verfahren zur Herstellung eines Peptids mit der in Figur 1 gezeigten Aminosäurensequenz, das mit anti-HCV-Antikörpern immunochemisch reaktiv ist, unter Anwendung der bekannten organisch chemischen Verfahren zur Peptidsynthese oder unter Anwendung rekombinanter DNA-Techniken.

3. Verfahren zur Herstellung eines Peptids mit der in Figur 5 gezeigten Aminosäurensequenz, das mit anti-HCV-Antikörpern immunochemisch reaktiv ist, unter Anwendung der bekannten organisch chemischen Verfahren oder unter Anwendung rekombinanter DNA-Techniken.

4. Verfahren zum Nachweis gegen HCV gerichteter Antikörper in einer Testflüssigkeit, dadurch gekennzeichnet, dass ein immunochemisches Reagenz, das ein Peptid mit der in einer der Figuren 1, 5 und 7 gezeigten Aminosäurensequenz umfasst, in Kontakt mit der Testflüssigkeit gebracht wird und die Anwesenheit von allen zwischen dem Peptid und den Antikörpern gebildeten Immunkomplexen in der Testflüssigkeit nachweist.

5. Verfahren zum Nachweis von HCV in einer Testflüssigkeit, dadurch gekennzeichnet, dass ein immunochemisches Reagenz, das ein Peptid mit der in einer der Figuren 1, 5 und 7 gezeigten Aminosäurensequenz umfasst, mit der Testflüssigkeit und anti-HCV-Antikörpern in Kontakt gebracht wird, wonach die Anwesenheit von gebildeten Immunkomplexen nachgewiesen wird, und die Anwesenheit von HCV in der Testflüssigkeit bestimmt wird.

## Revendications (Revendications pour l'(les) Etat(s) contractant(s) suivant(s): AT, BE, CH, DE, DK, FR, GB IT, LI)

1. Peptide réagissant immunochimiquement avec les anticorps anti-HCV, présentant la séquence d'acides aminés telle qu'indiquée dans la figure 7.

2. Peptide réagissant immunochimiquement avec les anticorps anti-HCV, présentant la séquence d'acides aminés telle qu'indiquée dans la figure 1.

3. Réactif immunochimique comprenant le peptide selon la revendication 2.

4. Peptide réagissant immunochimiquement avec les anticorps anti-HCV, présentant la séquence d'acides aminés telle qu'indiquée dans la figure 5.

5. Réactif immunochimique comprenant le peptide selon la revendication 4.

6. Un procédé de détection des anticorps dirigés contre HCV dans un fluide à tester, caractérisé en ce qu'un réactif immunochimique selon la revendication 3 ou 5 est mis en contact avec le fluide à tester et la présence des complexes immuns formés entre les peptides et les anticorps dans le fluide à tester est détectée.

7. Un procédé de détection du HCV dans un fluide à tester, caractérisé en ce qu'un réactif immunochimique selon la revendication 3 ou 5 est mis en contact avec le fluide à tester et les anticorps anti-HCV, puis la présence des complexes immuns formés est détectée et la présence du HCV dans le fluide à tester est déterminée.

8. Trousse de test pour la détection des anticorps anti-HCV selon le procédé de la revendication 6 ou 7.

## Revendications (Revendications pour l'(les) Etat(s) contractant(s) suivant(s): ES, GR)

1. Procédé de préparation d'un peptide réagissant immunochimiquement avec les anticorps anti-HCV, présentant la séquence d'acide aminé telle qu'indiquée dans la figure 7, utilisant l'une des méthodes chimiques organiques connues pour la synthèse peptidique ou utilisant des techniques d'ADN recombinant.

2. Procédé de préparation d'un peptide réagissant immunochimiquement avec les anticorps anti-HCV, présentant la séquence d'acide aminé telle qu'indiquée dans la figure 1, utilisant l'une des méthodes chimiques organiques connues pour la synthèse peptidique ou utilisant des techniques d'ADN recombinant.

3. Procédé de préparation d'un peptide réagissant immunochimiquement avec les anticorps anti-HCV, présentant la séquence d'acide aminé telle qu'indiquée dans la figure 5, utilisant l'une des méthodes chimiques organiques connues pour la synthèse peptidique ou utilisant des techniques d'ADN recombinant.

4. Un procédé de détection des anticorps dirigés contre HCV dans un fluide à tester, caractérisé en ce qu'un réactif immunochimique comprenant un peptide, présentant une séquence d'acide aminé telle qu'indiqué dans l'une quelconque des figures 1, 5 et 7, est mis en contact avec le fluide à tester et la présence de tout complexe immun formé entre le peptide et les anticorps dans le fluide à tester est détectée.

5. Un procédé de détection du HCV dans un fluide à tester, caractérisé en ce qu'un réactif immunochimique comprenant un peptide, présentant la séquence d'acide aminé telle qu'indiquée dans l'une quelconque des figures 1, 5 et 7, est mis en contact avec le fluide à tester et les anticorps anti-HCV, puis la présence des complexes immuns formés est détectée et la présence du HCV dans le fluide à tester est déterminée.
